Europäisches Patentamt

European Patent Office

Office européen des brevets

(19)

(11) Veröffentlichungsnummer: **0 043 039**
A2

(12) # EUROPÄISCHE PATENTANMELDUNG

(21) Anmeldenummer: **81104698.6**

(22) Anmeldetag: **19.06.81**

(51) Int. Cl.³: **C 07 C 11/12**
**C 07 C 2/74, B 01 J 31/24**

(30) Priorität: **01.07.80 DE 3024877**
**01.07.80 DE 3024878**

(43) Veröffentlichungstag der Anmeldung:
**06.01.82 Patentblatt 82/1**

(84) Benannte Vertragsstaaten:
**BE DE FR GB IT NL**

(71) Anmelder: **EC ERDÖLCHEMIE GMBH**
**Postfach 75 2002**
**D-5000 Köln 71(DE)**

(72) Erfinder: **Schleppinghoff, Bernhard, Dr.**
**Kolpingstrasse 5**
**D-4047 Dormagen 1(DE)**

(72) Erfinder: **Köhler, Hans-Dieter, Dr.**
**Ostpreussenallee 10**
**D-4047 Dormagen 1(DE)**

(72) Erfinder: **Schöneberger, Helmut, Dr.**
**Im Bergfeld 24**
**D-6729 Woerth/Rhein(DE)**

(74) Vertreter: **Mann, Volker, Dr. et al,**
**c/o Bayer Aktiengesellschaft Zentralbereich Patente**
**Marken und Lizenzen**
**D-5090 Leverkusen-Bayerwerk(DE)**

(54) **Verfahren zur Herstellung von 1,7-Octadien.**

(57) 1,7-Octadien wird durch Hydrodimerisation von 1,3-Butadien in Gegenwart eines Palladium-(II)-Salzes, eines tertiären Phosphins und eines organischen Lösungsmittels durch Umsetzung mit einem Gemisch aus Wasserstoff und Kohlendioxid als dem reduzierenden Stoff hergestellt. Es wird bei einem Druck von 5 bis 100 bar und einer Temperatur von 30 bis 150°C gearbeitet. Die Reaktion verläuft besonders vorteilhaft, wenn die Katalysatorlösung vor der Zugabe von 1,3-Butadien mit 0,1 bis 2000 Mol freier Ameisensäure pro Mol Palladium-Verbindung behandelt wird. Diese Vorbehandlung ist dann besonders günstig, wenn als Lösungsmittel ein eine Carbonylgruppe enthaltender N-Heterocyclus eingesetzt wird.

EP 0 043 039 A2

Croydon Printing Company Ltd.

EC Erdölchemie GmbH          Köln-Worringen

Ha/bc/c


Verfahren zur Herstellung von 1,7-Octadien
────────────────────────────────────────


Die vorliegende Erfindung betrifft ein Verfahren zur
Herstellung von 1,7-Octadien durch katalytische Hydrodimerisation von 1,3-Butadien.

Lineare, endständige Diolefine, wie das 1,7-Octadien,
sind wertvolle Zwischenprodukte, die in der Oxosynthese,
bei Hydrocyanierungen und zahlreichen anderen chemischen
Umsetzungen eingesetzt werden können. Der besondere Vorteil solcher Olefine, wie des 1,7-Octadiens, liegt in
der Möglichkeit, Diester, Dicarbonsäuren, Diole und
Diamine mit terminalen funktionellen Gruppen zu synthetisieren.

Aus US 3 823 199, US 3 732 328 und GB 1 341 324 ist
es bereits bekannt, 1,3-Butadien in homogener Phase
in Gegenwart von Palladium-(II)-Salzen und tertiären
Phosphinen durch einen reduzierenden Stoff zu hydodimerisieren, wobei 1,7-Octadien in wechselnden Mengen neben 1,6-Octadien und anderen Umwandlungsprodukten des Butadiens entsteht. In den genannten Patentschriften wird als reduzierender Stoff bevorzugt


EC 109-Ausland
─────────────

Ameisensäure verwendet.

Neben der Ameisensäure sind auch andere reduzierende Stoffe zur Hydrodimerisierung von 1,3-Butadien eingesetzt worden, wie Hydrazin, Formaldehyd, niedere Alkohole oder Wasserstoff (GB 1 341 324). In der europäischen Patentanmeldung 0 004 408 wird als reduzierender Stoff das Triethylammoniumsalz der Ameisensäure verwendet, da ein über die Dissoziation hinaus vorhandener Überschuß freier Ameisensäure die Reaktion inhibieren soll. Zur Promotion der Hydrodimerisierung wird in dieser Patentanmeldung weiter vorgeschlagen, vor der Zugabe des 1,3-Butadiens den Katalysator durch Zugabe des Ammoniumsalzes der Ameisensäure oder durch Wasserstoff 1 Stunde lang bei 60°C vorzubehandeln.

Keine der genannten Patentschriften zeigt, daß in Gegenwart von Palladium-(II)-Salzen und Wasserstoff als dem reduzierenden Stoff eine erfolgreiche Hydrodimerisierung des 1,3-Butadiens zum 1,7-Octadien vorgenommen wurde. US 3 732 328 zeigt im Beispiel 7 die Verwendung von Wasserstoff als reduzierenden Stoff, wobei allerdings in Gegenwart von Rutheniumtrichlorid gearbeitet wird und als Hydrodimerisierungsprodukt das 1,6-Octadien erhalten wird. Eine Nacharbeitung dieses Ausführungsbeispiels unter Verwendung von Palladiumacetat und Triphenylphosphin, das nach US 3 732 328 zur Bildung eines Gemisches aus 1,6- und 1,7-Octadien führen soll, in verschiedenen Lösungsmitteln ergab keine Hydrodimerisation des 1,3-Butadiens.

EC 109

Es wurde nun ein Verfahren zur Herstellung von 1,7-Octadien durch Hydrodimerisation von 1,3-Butadien in Gegenwart eines Palladium-(II)-Salzes, eines tertiären Phosphins, eines reduzierenden Stoffes und eines organischen Lösungsmittels gefunden, das dadurch gekennzeichnet ist, daß man als reduzierenden Stoff ein Gemisch von Wasserstoff und Kohlendioxid bei einem Druck von 5 bis 100 bar einsetzt und bei einer Temperatur von 30 bis 150°C arbeitet und gegebenenfalls die Katalysatorlösung, die die Pd-(II)-Verbindung und das tertiäre Phosphin enthält, vor der Zugabe von 1,3-Butadien mit 0,1 bis 2000 Mol freier Ameisensäure pro Mol Pd-Verbindung behandelt.

Im erfindungsgemäßen Verfahren wird als reduzierender Stoff ein Gemisch aus Wasserstoff und Kohlendioxid eingesetzt, wobei der Gesamtdruck, unter dem das Reaktionsgemisch liegt, 5 bis 100 bar, vorzugsweise 10 bis 30 bar, beträgt und der Wasserstoff- bzw. der Kohlendioxid-Partialdruck unabhängig voneinander jeweils 2 bis 80 bar, vorzugsweise 4 bis 20 bar, beträgt. Das Verhältnis der Partialdrucke von Wasserstoff und Kohlendioxid kann hierbei in weiten Grenzen schwanken. Beispielsweise sei ein Verhältnis der Partialdrucke von Wasserstoff zu Kohlendioxid wie 1:0,02 bis 3, bevorzugt 1:0,03 bis 2, genannt. Dies entspricht etwa einem Molverhältnis von 0,02-2,5 Mol, bevorzugt 0,03-2 Mol $CO_2$ pro Mol $H_2$. Besonders bevorzugt sind 0,2-1 Mol $CO_2$ pro Mol $H_2$.

Es wurde weiterhin gefunden, daß mit zunehmendem Kohlendioxid-Partialdruck der Umsatz und die Selektivität im erfindungsgemäßen Verfahren zunehmen, während ein steigender Wasserstoff-Partialdruck das Gegenteil

EC 109

- 4 -

bewirken kann. Weiterhin können mit steigendem Gesamtdruck im Reaktionssystem Umsatz und Selektivität etwas
zurückgehen.

Das erfindungsgemäße Verfahren wird bei einer Temperatur von 30 bis 150°C, vorzugsweise 60 bis 110°C,
durchgeführt.

Als Palladium-(II)-Salze für das erfindungsgemäße
Verfahren seien beispielsweise das Chlorid, das Bromid, das Jodid, das Nitrat, das Acetat, das Propionat,
das Butyrat, das Acetylacetonat, bevorzugt das Acetat,
genannt.

Es wird beispielsweise ein Gewichtsverhältnis von Butadien zu Palladiumsalz von 250 bis 2000:1, vorzugsweise 300 bis 350:1, eingestellt.

Als tertiäres Phosphin für das erfindungsgemäße Verfahren sei beispielsweise ein Trialkylphosphin, wie
Tripropylphosphin, Triisopropylphosphin, Tributylphosphin, Triisobutylphosphin, Trioctylphosphin oder
Trihexylphosphin, oder ein Triarylphosphin, wie Triphenylphosphin, Tri-p-tolylphosphin, Tri-p-methoxyphenyl-phosphin, bevorzugt Triphenylphosphin, genannt.
Es wird beispielsweise ein Gewichtsverhältnis von
Butadien zu dem tertiären Phosphin wie 100 bis 1000:1,
vorzugsweise 130 bis 160:1, eingestellt.

Das erfindungsgemäße Verfahren wird in homogener,
flüssiger Phase in einem organischen Lösungsmittel

EC 109

durchgeführt, wobei beispielsweise in einem Rührkessel oder einem Strömungsrohr als Reaktionsapparat
gearbeitet werden kann. Als organische Lösungsmittel
für das erfindungsgemäße Verfahren kommen sowohl
unpolare als auch polare Lösungsmittel in Frage,
die sich unter den Reaktionsbedingungen nicht verändern. Als nicht-polares Lösungsmittel sei beispielsweise Hexan, Octan, Dodecan, Benzol, Toluol, Xylol,
Cyclohexan oder Methylcyclohexan genannt. Als polare
Lösungsmittel seien beispielsweise Nitrile, wie Benzonitril oder Acetonitril, Sulfoxide, wie Dimethylsulfoxid, Sulfone wie Tetramethylensulfon, Ester
wie Benzylacetat oder Dimethylphthalat, Ether, wie
Diisopropylether, Dioxan oder Tetrahydrofuran, Mono-
oder Diethylenglykol, tertiäre Amine, wie Triethylamin, N,N-Dimethylanilin oder Pyridin, Amide, wie
Acetamid, Benzamid, N-Methyl-acetamid, Dimethylformamid, oder Dimethylacetamid, oder sauerstoffhaltige
N-Heterocyclen, wie Pyrrolidon, Piperidon, N-Methyl-
pyrrolidon, N-Methyl-caprolactam, N-Methyl-piperidon,
N-Ethyl-piperidon, N-ß-Dimethyl-propiolactam oder
N-Ethyl-ß-methylpropiolactam, genannt.

Es können auch Gemische von zwei oder mehreren der genannten Lösungsmittel eingesetzt werden. Unter den genannten Lösungsmitteln werden bevorzugt polare organische Lösungsmittel, besonders bevorzugt sauerstoffhaltige N-Heterocyclen und ganz besonders bevorzugt
das N-Methyl-pyrrolidon im erfindungsgemäßen Verfahren eingesetzt. Hierbei wird ein Gewichtsverhältnis von Lösungsmittel zu Butadien wie 0,5 bis 5:1,
vorzugsweise 1,5 bis 3,5:1, eingestellt.

EC 109

Man kann das erfindungsgemäße Verfahren auch in Gegenwart inerter Gase, wie Argon, Stickstoff oder Methan, durchführen.

Eine besondere Verfahrensvariante stellt die erfindungsgemäße Vorbehandlung mit freier Ameisensäure dar, besonders wenn als Katalysatorlösung eine Lösung von Palladium-(II)-acetat und Triphenylphosphin in einem eine Carbonylgruppe enthaltenden N-Heterocyclus vorliegt.

Überraschenderweise und entgegen den Feststellungen des Standes der Technik ist eine Vorbehandlung des Katalysators mit freier, nicht salzartig gebundener Ameisensäure ohne Inhibierung der Hydrodimerisierungsreaktion möglich, so daß die Hydrodimerisierung anschließend mit Wasserstoff durchgeführt werden kann, dem die genannte Menge Kohlendioxid zugemischt wurde. Dieser überraschende Effekt ist besonders groß, wenn in N-Methyl-pyrrolidon als Lösungsmittel gearbeitet wird.

Die freie, nicht salzartig gebundene Ameisensäure wird im molaren Verhältnis von 0,1 bis 2000 pro Mol Palladium-Verbindung verwendet. Bevorzugt ist die Verwendung von 1 bis 1500 Mol Ameisensäure pro Mol Palladium-Verbindung. Besonders bevorzugt ist eine Menge von 1 bis 150 Mol Ameisensäure pro Mol Palladium-Verbindung.

Die Katalysatorvorbehandlung erfolgt bei 0 bis 60°C, bevorzugt bei 20 bis 30°C, besonders bevorzugt bei etwa 25°C.

EC 109

- 7 -

Zur erfindungsgemäßen Vorbehandlung reicht eine Zeit
von etwa 1 Minute. Als längste Vorbehandlungszeit
sei etwa ein Zeitraum von 60 Minuten angegeben. Längere Vorbehandlungszeiten sind unkritisch, jedoch unwirtschaftlich. Bevorzugt wird die Vorbehandlung während einer Zeit von 3 bis 8 Minuten durchgeführt.

Das 1,7-Octadien kann beispielsweise durch Destillation
aus dem Reaktionsgemisch isoliert werden. Die als Sumpfphase einer solchen Destillation verbleibende Katalysatorlösung, die die Palladium-Verbindung, das tertiäre
Phosphin und das Lösungsmittel enthält, kann für weitere
Reaktionsansätze im erfindungsgemäßen Verfahren verwendet werden. Gegebenenfalls wird ein Teil dieser
Katalysatorlösung, beispielsweise 0,1 bis 10 Gew.-%,
ausgeschleust und durch die Palladium-Verbindung, das
tertiäre Phosphin und Lösungsmittel in der entsprechenden Konzentration ersetzt, um den Gehalt an Nebenprodukten in der Katalysatorlösung zu senken.

Gegenüber den in den bekannten 1,7-Octadien-Herstellungsverfahren verwendeten reduzierenden Stoffen liegen bei
der Verwendung von Gemischen aus Wasserstoff und Kohlendioxid die Vorteile in der besseren Verfügbarkeit
dieser Stoffe, in ihren geringeren Kosten und in dem
geringeren Anspruch an die Materialgüte der Reaktionsapparaturen.

Es ist überraschend, daß durch die Zumischung von Kohlendioxid zum Wasserstoff bei der Anwendung auf die
Hydrodimerisation des 1,3-Butadiens als Reaktionspro-

EC 109

dukt 1,7-Octadien erhalten werden kann, während nach dem Stande der Technik bei Verwendung von Wasserstoff als reduzierendem Stoff keine Hydrodimerisation erfolgt oder in geringer Menge das 1,6-Octadien nachgewiesen werden kann.

EC 109

- 9 -

Vergleichsbeispiel 1-4 und Beispiele 5-12

Ein stopfbuchsloser 300-ml-V4A-Stahlautoklav mit Magnetrührwerk, Druck- und Temperatur-Regelung und mit
Stickstoff inertisiert, wurde mit einer Katalysatorlösung, bestehend aus $4 \cdot 10^{-4}$ Mol Pd-(II)-acetat, $7,78 \cdot$
$10^{-4}$ Mol Triphenylphosphin in dem nach Art und Menge
in der Tabelle I angegebenen Lösungsmittel und $5,5 \cdot 10^{-1}$
Mol 1,3-Butadien beschickt.

Über eine Druckhaltung wurde das $H_2/CO_2$-Mischgas (Beispiele 5 bis 12) bzw. der reine $H_2$ (Beispiele 1 bis 4)
in den Mengen, die in der Tabelle I angeführt sind,
dem Autoklaven über die Reaktionszeit kontinuierlich
zudosiert.

Der Reaktionsdruck wurde bei den in der Tabelle I angegebenen Werten konstant gehalten. Die Reaktionstemperatur wurde über die Reaktionszeit von 0,5 Stunden
in den Grenzen von 75 bis 90°C geregelt.

Die Zusammensetzung des Reaktionsproduktes wurde gaschromatographisch bestimmt, so daß die in der Tabelle I
angeführten Butadienumsätze und Selektivitäten zum
1,7-Octadien ermittelt wurden.

EC 109

Tabelle I

| Bei-spiel | Lösungs-mittel | Reaktions-druck | $H_2$ | $CO_2$ | Umsatz des Butadiens | Selektivität zu 1,7-Octadien |
|---|---|---|---|---|---|---|
| | 100 ml | bar | mol | mol | % | % |
| 1 | [+]DMF/$Et_3$N | 18 | $2,76.10^{-1}$ | – | nur Hydrierung | – |
| 2 | [+]Benzol/ $Et_3$N | 18 | " | – | " | – |
| 3 | [+]Cyclohexan $Et_3$N | 18 | " | – | " | – |
| 4 | NMP | 18 | " | – | " | – |
| 5 | NMP | 18 | " | $0,08.10^{-1}$ | 7,3 | 60,0 |
| 6 | NMP | 18 | " | $0,24.10^{-1}$ | 8,0 | 58,0 |
| 7 | NMP | 18 | " | $0,40.10^{-1}$ | 14,0 | 62,0 |
| 8 | NMP | 18 | " | $0,80.10^{-1}$ | 20,0 | 64,5 |
| 9 | NMP | 18 | " | $2,76.10^{-1}$ | 42,0 | 68,5 |
| 10 | NMP | 30 | " | $0,40.10^{-1}$ | 10,0 | 57,0 |
| 11 | NMP | 30 | " | $0,80.10^{-1}$ | 12,0 | 57,0 |
| 12 | NMP | 70 | " | $2,76.10^{-1}$ | 35,0 | 33,0 |

DMF = Dimethylformamid; $Et_3$N = Triethylamin; NMP = N-Methyl-pyrrolidon; [+] Molverhältnis
Lösungsmittel: $Et_3$N = 2,5:1

Beispiele 13-19

Ein stopfbuchsloser 300-ml-V4A-Stahlautoklav mit Magnetrührwerk, Druck- und Temperaturregelung wurde mit einer Katalysatorlösung, bestehend aus:

$5,4 \cdot 10^{-4} - 5,4 \cdot 10^{-1}$ Mol HCOOH (entsprechend den Angaben der Tabelle II),

$4 \cdot 10^{-4}$ Mol Pd-acetat, $7,78 \cdot 10^{-4}$ Mol Triphenylphosphin und 10,1 Mol N-Methyl-pyrrolidon (NMP)

beschickt.

Nach 5 Minuten bei 25°C wurden $5,5 \cdot 10^{-1}$ Mol 1,3-Butadien zugefügt. Das $H_2/CO_2$-Mischgas (Konzentration s. Tabelle II) wurde über eine Druckhaltung dem Autoklaven während der Reaktionszeit von 0,5 Stunden kontinuierlich zudosiert.

Die Reaktionstemperatur wurde während der Reaktionszeit in den Grenzen von 75 bis 90°C geregelt. Der Reaktionsdruck wurde bei 25 bar gehalten.

Die Zusammensetzung des Reaktionsproduktes wurde gaschromatographisch bestimmt, so daß die in der Tabelle II angeführten Butadien-Umsätze und Selektivitäten zum 1,7-Octadien ermittelt wurden.

EC 109

Tabelle II

| Beispiel | HOOOH (Mol) | Lösungs-mittel | Mischgas $H_2$ (Mol) | $CO_2$ (Mol) | Umsatz an Butadien (%) | Selektivität zum 1,7-Octadien (%) |
|---|---|---|---|---|---|---|
| 13 | $5,4.10^{-1}$ | NMP | $2,48.10^{-1}$ | $2,48.10^{-1}$ | 60 | 57 |
| 14 | $5,4.10^{-2}$ | NMP | $2,73.10^{-1}$ | $2,73.10^{-1}$ | 51 | 60 |
| 15 | $5,4.10^{-3}$ | NMP | $2,76.10^{-1}$ | $2,76.10^{-1}$ | 54 | 60 |
| 16 | $5,4.10^{-4}$ | NMP | $2,76.10^{-1}$ | $2,76.10^{-1}$ | 40 | 43 |
| 17 | $5,4.10^{-3}$ | NMP | $2,76.10^{-1}$ | $1,84.10^{-1}$ | 56 | 62 |
| 18 | $5,4.10^{-3}$ | NMP | $2,76.10^{-1}$ | $1,38.10^{-1}$ | 50 | 70 |
| 19 | $5,4.10^{-3}$ | NMP | $2,76.10^{-1}$ | $0,91.10^{-1}$ | 51 | 64 |

Patentansprüche

1) Verfahren zur Herstellung von 1,7-Octadien durch Hydrodimerisation von 1,3-Butadien in Gegenwart eines Palladium-(II)-Salzes, eines tertiären Phosphins, eines reduzierenden Stoffes und eines organischen Lösungsmittels, dadurch gekennzeichnet, daß man als reduzierenden Stoff ein Gemisch von Wasserstoff und Kohlendioxid bei einem Druck von 5 bis 100 bar einsetzt und bei einer Temperatur von 30 bis 150°C arbeitet und gegebenenfalls die Katalysatorlösung, die die Pd-(II)-Verbindung und das tertiäre Phosphin enthält, vor der Zugabe von 1,3-Butadien mit 0,1 - 2000 Mol freier Ameisensäure pro Mol Pd-verbindung behandelt.

2) Verfahren nach Anspruch 1, dadurch gekennzeichnet, daß der Wasserstoff-Partialdruck und der Kohlendioxid-Partialdruck unabhängig voneinander innerhalb des Gesamtdruckes 2 bis 80 bar beträgt.

3) Verfahren nach Ansprüchen 1 und 2, dadurch gekennzeichnet, daß der Gesamtdruck im Bereich von 10 bis 30 bar gehalten wird.

4) Verfahren nach Ansprüchen 1 bis 3, dadurch gekennzeichnet, daß das Verhältnis der Partialdrücke von Wasserstoff zu Kohlendioxid wie 1:0,02 bis 3 beträgt.

EC 109

5) Verfahren nach Ansprüchen 1 bis 4, dadurch gekennzeichnet, daß das Verhältnis der Partialdrucke von Wasserstoff zu Kohlendioxid wie 1:0,03 bis 2 beträgt.

6) Verfahren nach Ansprüchen 1 bis 5, dadurch gekennzeichnet, daß man bei einer Temperatur von 60 bis 110°C arbeitet.

7) Verfahren nach Ansprüchen 1 bis 6, dadurch gekennzeichnet, daß man die Behandlung der Katalysatorlösung mit Ameisensäure durchführt, wenn ein eine Carbonylgruppen enthaltender N-Heterocyclus eingesetzt wird.

8) Verfahren nach Ansprüchen 1 bis 7, dadurch gekennzeichnet, daß die Katalysatorbehandlung bei 0 bid 60°C erfolgt.

9) Verfahren nach Ansprüchen 1 bis 8, dadurch gekennzeichnet, daß eine Katalysatorlösung, die Palladium-(II)-acetat und Triphenylphosphin in N-Methylpyrrolidon als Lösungsmittel enthält, mit freier Ameisensäure vorbehandelt wird.

10) Verfahren nach Ansprüchen 1 bis 9, dadurch gekennzeichnet, daß zur Hydrodimerisation am vorbehandelten Katalysator Wasserstoff eingesetzt wird, dem 0,1 bis 1 Mol Kohlendioxid pro Mol Wasserstoff zugemischt werden.

EC 109